# EUROPEAN PATENT SPECIFICATION

(11) **EP 3 833 354 B1**
(45) Date of publication and mention of the grant of the patent: **04.10.2023**
(21) Application number: 19752472.1
(22) Date of filing: 06.08.2019
(51) Int. Cl.: A61K 31/519, A61P 9/10, A61P 9/14, A61P 9/08, A61P 9/12

(54) **TISSUE TRANSGLUTAMINASE MODULATORS FOR MEDICINAL USE**
GEWEBETRANSGLUTAMINASEMODULATOREN ZUR MEDIZINISCHEN VERWENDUNG
MODULATEURS DE LA TRANSGLUTAMINASE TISSULAIRE POUR UTILISATION MÉDICALE

(30) Priority: 07.08.2018 EP 18187711
(43) Date of publication of application: 16.06.2021
(73) Proprietor: Aarhus Universitet, 8000 Aarhus C (DK)
(72) Inventor: PÉREZ, Estéfano, Pinilla,, 8240 Risskov (DK); SIMONSEN, Ulf, 8380 Trige (DK)
(74) Representative: Plougmann Vingtoft a/s
(86) International application number: PCT/EP2019/071134
(87) International publication number: WO 2020/030648

(56) References cited:
- WO-A1-2006/060702
- MATLUNG HANKE L ET AL: "Transglutaminase activity regulates atherosclerotic plaque composition at locations exposed to oscillatory shear stress", ATHEROSCLEROSIS, vol. 224, no. 2, 2012, pages 355-362, XP028942294, ISSN: 0021-9150, DOI: 10.1016/J.ATHEROSCLEROSIS.2012.07.044
- MORTEN ENGHOLM ET AL: "Involvement of transglutaminase 2 and voltage-gated potassium channels in cystamine vasodilatation in rat mesenteric small arteries : Vasoactive effects of cystamine", BRITISH JOURNAL OF PHARMACOLOGY, vol. 173, no. 5, 27 January 2016 (2016-01-27), pages 839-855, XP055551401, UK ISSN: 0007-1188, DOI: 10.1111/bph.13393
- H TATSUKAWA ET AL: "Transglutaminase 2 has opposing roles in the regulation of cellular functions as well as cell growth and death", CELL DEATH & DISEASE, vol. 7, no. 6, 1 June 2016 (2016-06-01), pages e2244-e2244, XP055552630, DOI: 10.1038/cddis.2016.150

## Description

### Technical field of the invention

The present invention relates to compounds which modulate tissue transglutaminase (TG2) for use in the treatment of endothelial dysfunction and diseases related thereto. In particular the present invention relates to the compound LDN-27219 (formula II herein) and derivatives thereof for use in the treatment of diseases resulting from endothelial dysfunction, especially in association with aging and/or diabetes.

### Background of the invention

The endothelium is a cellular monolayer that coats the luminal surface of the vascular system and plays an essential role in the regulation of vascular tone in response to hemodynamics and chemical signals. The functioning of the endothelial cells is influenced by Tissue Transglutaminase (TG2), which is an enzyme of the transglutaminase family (TG1-7 and Factor XIII). The TG2 enzyme is highly expressed in endothelial and smooth muscle cells. TG2 is located in different intracellular and extracellular compartments and has several functions depending on the location and conformation of the enzyme.

TG2 is found in the body in a closed conformation and in an open confirmation. When TG2 is bound to Ca²⁺, it conforms to an open conformation that shows extracellular transamidase activity and leads to covalent cross-linking of structural proteins. In the closed conformation, it presents intracellular GTP-binding activity that enables it to act as a G protein (Gna).

Elevated levels and/or activity of TG2 is associated with a number of diseases including cancer, neurodegenerative diseases, fibrosis, and celiac diseases, and therefore, different types of TG2 inhibitors have been developed and used to investigate how TG2 inhibition may be used for treatment of these diseases. A plethora of inhibitors have been investigated, including compounds such as cystamine and LDN-27219.

Generally, these studies have however not studied the effects of using compounds which inhibit the open conformation by promoting the closed conformation of TG2, and no compounds have been identified for effective treatment of endothelial dysfunction, particularly in association with secondary conditions including old-age or diabetes, which influence TG2 expression.

Thus, M. Engholm et al., British J. Pharm. (2016), 173, 839-855 discloses primarily a study of the vasodilatory effects of cystamine in mesenteric small arteries in rats. The study uses LDN-27219 as a comparative TG2 inhibitor and concluded that both compounds have the ability to relax contracted arteries in rats. The study does not disclose the promotion of the closed conformation TG2 or any beneficial effects hereof, nor age- or diabetes related effects of the TG2 inhibitors therein.

Y.J. Oh et al., Amino Acids, 2017, 49(3), 695-704 discloses the role of TG2 in age-associated ventricular stiffness. Cystamine, a known general TG2 inhibitor, improves the cardiac function in aging rats. The use of TG2 modulators promoting the closed confirmation of TG2, including LDN-27219, is not disclosed.

Thus, J. W. Keillor et al., Trends in pharmacological sciences, 2015, Vol 36, no 1 discloses a number of inhibitors of TG2 and their effects. TG2 is described as related to particularly celiac disease, fibrosis and cancer. LDN-27219 is disclosed as an allosteric reversible slow binding inhibitor of TG2. The efficacy of LDN-27219 in any particular diseases is not disclosed, and neither is activity relation to endothelial dysfunction. The document discloses the comparative compounds used herein, i.e. Boc-DON and Z-DON (Z013 and Z006 therein respectively).

US 2018/0002700 A1 discloses the use of TG2 inhibitors, such as LDN-27219 for the treatment of diabetic complications caused by vascular leakage (claim 1). There are no data related to LDN-27219. Age and diabetes-related effects of LDN-27219, or any other TG2 inhibitors, are not disclosed or any relation thereof to promotion of the closed conformation of TG2.

WO 2014/057266 discloses the use of specific TG2 inhibitors in the treatment of age-related macular degeneration. LDN-27119 and treatment of age- or diabetes related endothelial dysfunction is not disclosed.

US 8,614,233 discloses cinnamoyl inhibitors of TG2, and links TG2 with a long list of diseases. LDN-27219 and derivatives are not disclosed.

Hence, an improved control of the effects of TG2 modulator control over the conformation of the enzyme would be advantageous, and in particular a more efficient inhibitor of the open conformation TG2 and promoter of the closed conformation TG2, useful for treatment of endothelial dysfunction, particularly in subjects of old-age or subjects diagnosed with diabetes, would be advantageous.

### Summary of the invention

The present invention thus uses reversible TG2 modulators as defined in claim 1, such as LDN-27219, to stabilize the closed conformation of the enzyme TG2. This leads to e.g. relaxation of arteries, improvement of endothelium-derived vasodilation and antihypertensive effects, representing a new pathway for the treatment of diseases related to endothelial dysfunction.

Thus, an object of the present invention relates to the provision of an improved treatment of endothelial dysfunction, particularly in aging and diabetic subjects.

In particular, it is an object of the present invention to provide a treatment of endothelial dysfunction and diseases related thereto, that solves the above mentioned problems of the prior art with TG2 inhibitors as defined in claim 1 that are non-selective and have unknown effect in the conformation of the enzyme or those that are selective, but lock the enzyme in the open conformation. These inhibitors are less effective in the treatment of endothelial dysfunction and more likely to induce undesirable side-effects, particularly in aging and diabetic subjects.

Thus, a first aspect of the present invention relates to a compound which is a modulator of tissue transglutaminase (TG2) for use in the treatment of endothelial dysfunction, wherein the modulator of tissue transglutaminase (TG2) is a compound of formula (I): 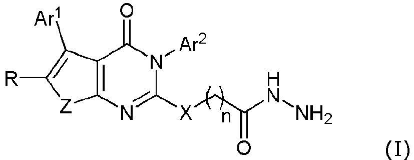 wherein
- Z is selected from the group consisting of S, SO₂, and Se,
- Ar¹ and Ar² are independently selected from the group consisting of optionally substituted phenyl and optionally substituted heteroaryl,
- R is selected from the group consisting of hydrogen, deuterium, halogen, and C₁-C₃ alkyl,
- X is selected from the group consisting of O, NH, N(Me), and S,
n is an integer selected from the group consisting of 1, 2 and 3.

A second aspect of the invention relates to a method of treating endothelial dysfunction comprising administering to a subject in need thereof a compound which is a modulator of tissue transglutaminase (TG2) as defined in claim 1.

The present inventors have surprisingly found that modulators of TG2, which inhibit the open conformation of the TG2 enzyme while promoting the closed conformation, such as LDN-27219 and derivatives, are effective in the treatment of endothelial dysfunction and diseases related thereto. Particularly, these compounds are effective in aging and diabetic subjects, where a pronounced effect is seen as compared to control and also compared to TG2 inhibitors that lock the enzyme in its open conformation, or that inhibit the enzyme more generally and/or irreversibly.

### Brief description of the figures

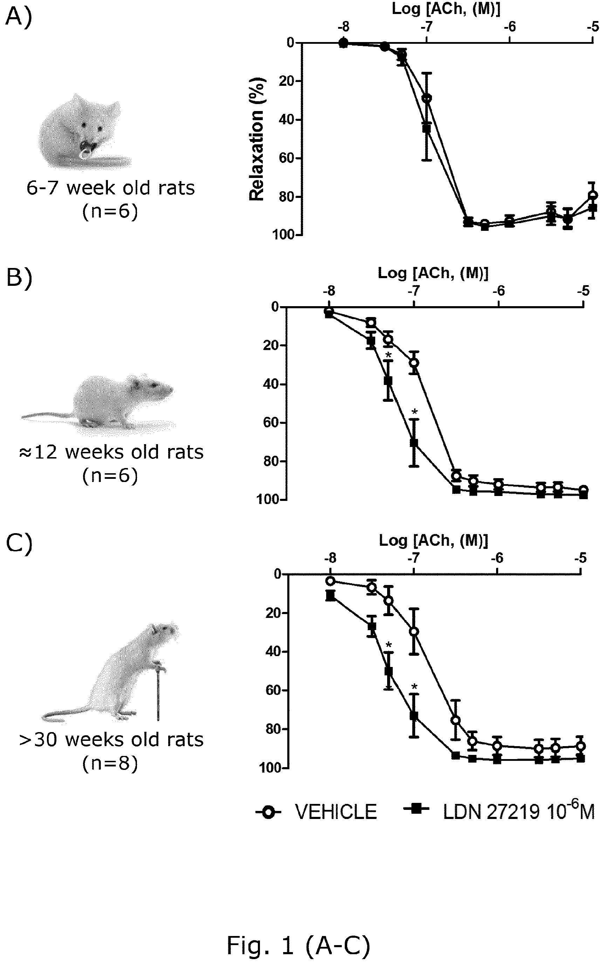

**Figure 1** shows that treatment with LDN-27219 (10⁻⁶ mol/L) improves the endothelium-derived relaxation of mesenteric arteries extracted from Male Wistar rats as induced by acetylcholine (10⁻⁸-10⁻⁵ mol/L) as compared to the control experiments (vehicle), and that the effect of LDN-27219 is particularly pronounced in older rats. Thus, Figure 1 A) shows relaxation % of the mesenteric arteries in healthy 6-7 week old rats as a function of acetylcholine concentration. Figure 1 B) shows relaxation % of the mesenteric arteries in healthy ≈12 week old rats. Figure 1 C) shows relaxation % of the mesenteric arteries in healthy >30 week old rats.

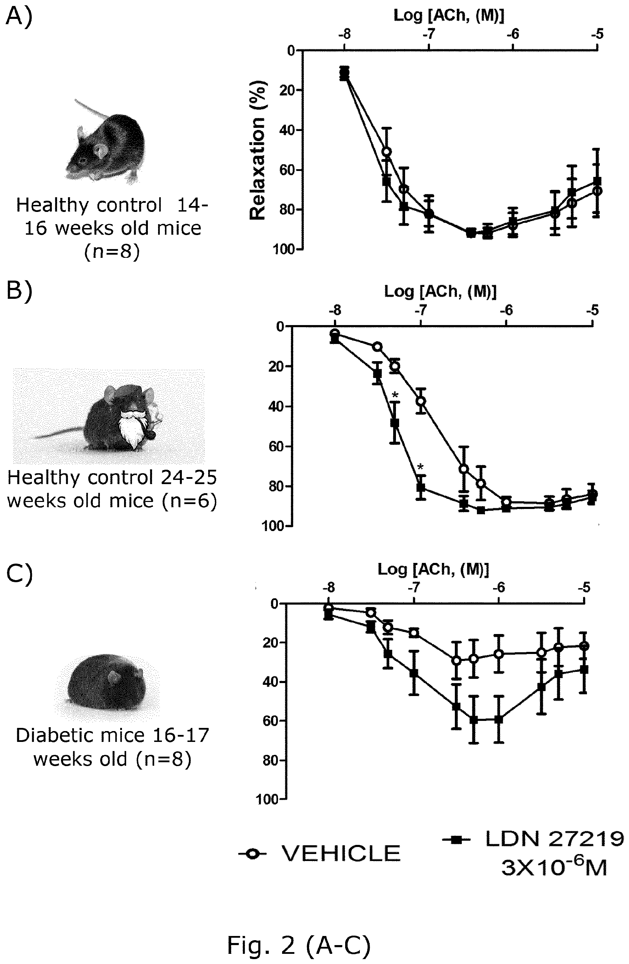

**Figure 2** shows that LDN-27219 (3·10⁻⁶ mol/L) improves the endothelium-derived relaxation of small mesenteric arteries extracted from male BKS diabetic mice as induced by acetylcholine (10⁻⁸-10⁻⁵ mol/L) as compared to the control experiments (vehicle), and that the effect of LDN-27219 is particularly pronounced in older and diabetic mice. Thus, Figure 2 A) shows relaxation % of the mesenteric arteries in 14-16 week old healthy control BKS mice that do not express type 2 diabetes as a function of acetylcholine concentration. Figure 2 B) shows relaxation % of the mesenteric arteries in 24-25 week healthy control BKS mice that do not express type 2 diabetes. Figure 2 C) shows relaxation % of the mesenteric arteries in 16-17 week old mice with diabetes 2.

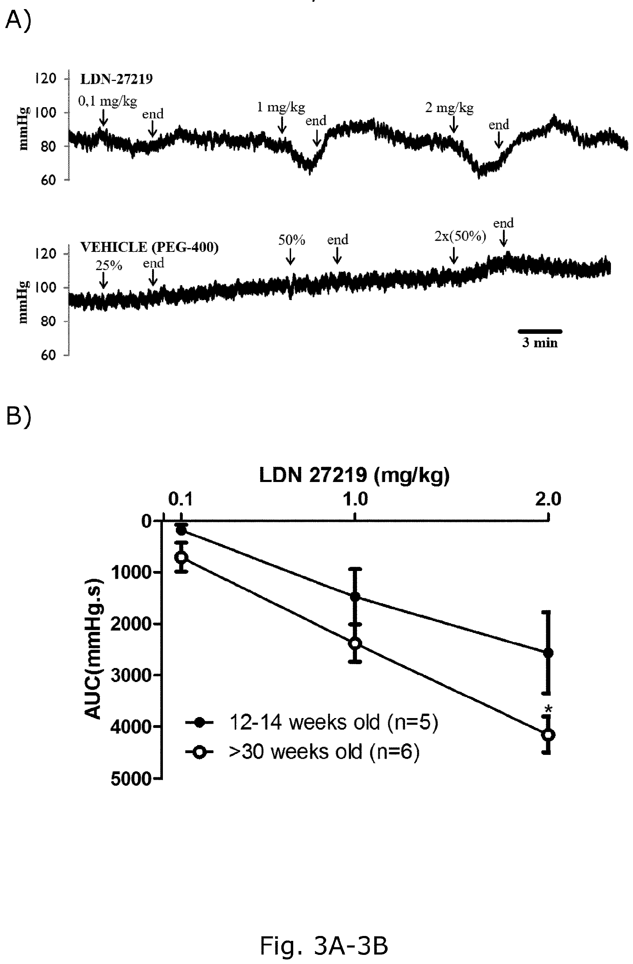

**Figure 3 A)** Shows that the infusion of three doses of LDN-27219 (0.1, 1.0 and 2.0 mg/kg) each lower the mean blood pressure (mmHg) in the carotid artery (antihypertensive effect), as well as a control experiment infusing the vehicle PEG-400.
**Figure 3 B)** Shows the calculated integral of the blood pressure (AUC) for the three additions of LDN-27219 shown in figure 3 A) and illustrates the increased anti-hypertensive effect in older rats (> 30 weeks old) as compared to younger rats (12-14 weeks old).

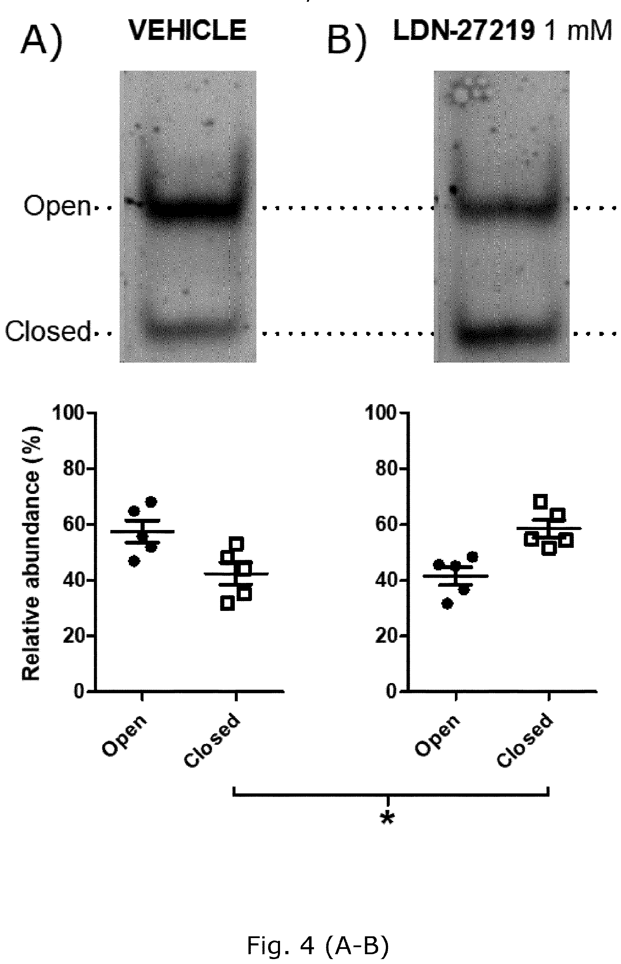

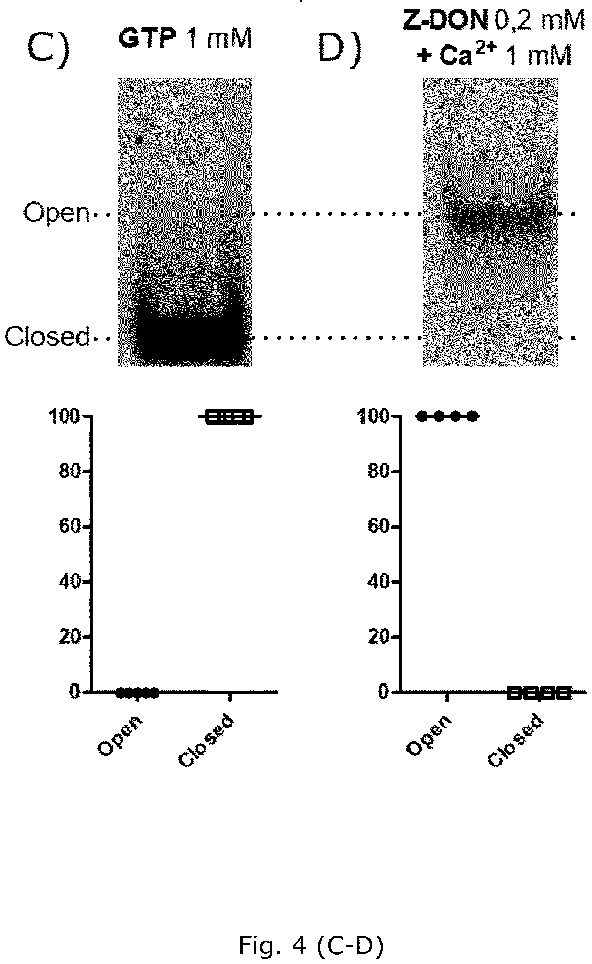

**Figure 4****)** Shows that LDN-27219 (10⁻³ mol/L) is able to increase the percentage of purified human TG2 in the closed conformation as compared to control conditions (vehicle). Thus, Figure 4 A) shows the two bands corresponding to the open and closed conformations (above) and their relative abundance in percentage (below) in control conditions. Figure 4 B) shows the two bands corresponding to the open and closed conformations (above) and their relative abundance in percentage (below) in presence of LDN-27219 (10⁻³ mol/L). Figure 4 C) shows the band corresponding to the closed conformation (above) and its relative abundance in percentage (below) in presence of GTP (10⁻³ mol/L), natural ligand of TG2 that is known to force the closed conformation of the enzyme. Figure 4 D) shows the band corresponding to the open conformation (above) and its relative abundance in percentage (below) in presence of Z-DON (0.2·10⁻³ mol/L) and Ca²⁺ (10⁻³ mol/L), ligands of TG2 that are known to force the open conformation of the enzyme.

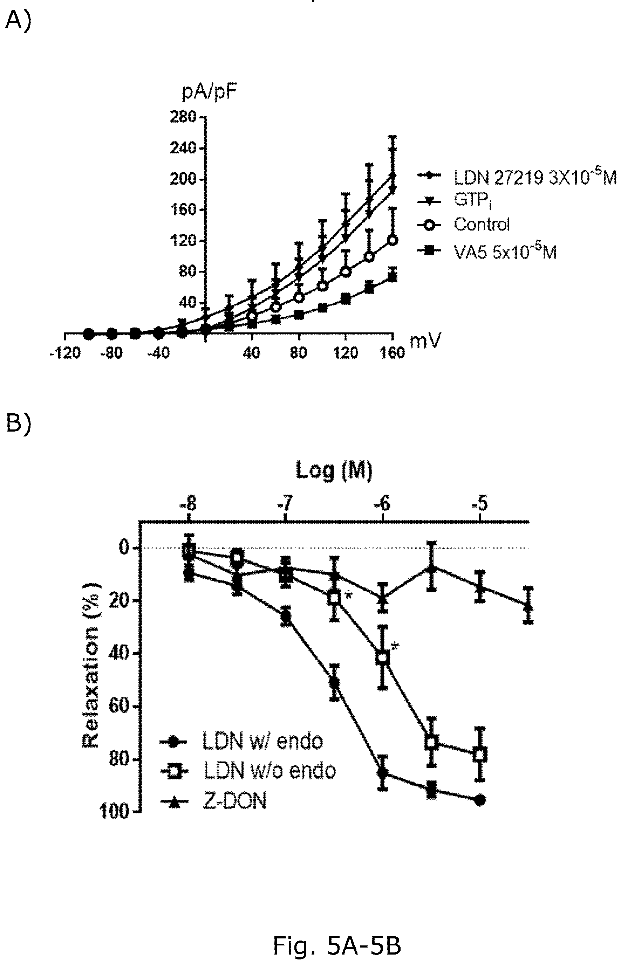

**Figure 5 A)** Shows the average of the current density(pA/pF)-voltage(mV) relationships of isolated vascular smooth muscle cells (VSMCs) exposed to different TG2 modulators. LDN-27219 (3·10⁻⁵ mol/L) increased potassium currents compared to the control conditions in an equivalent way to intracellular GTP, which has been shown to induce TG2 closed conformation, while (S)-Benzyl (6-acrylamido-1-(4-(7-hydroxy-2-oxo-2H-chromene-3-carbonyl)piperazin-1-yl)-1-oxohexan-2-yl)carbamate) (from now on VA-5) (5·10⁻⁵ mol/L), a TG2 irreversible inhibitor that has been shown to lock TG2 into the open conformation decreased potassium currents on these cells.
**Figure 5 B)** Shows the different effects of TG2 inhibitors on the tension of small mesenteric arteries extracted from ≈12 week old Male Wistar rats depending on their effect on the conformation of the enzyme. LDN-27219 (10⁻⁸-10⁻⁵ mol/L), that is believed to promote the closed conformation of TG2, induces a direct and dose-dependent relaxation of the arteries, which is sensitive to the removal of the endothelial layer. On the other hand, Z-DON-Val-Pro-Leu-OMe (from now on Z-DON) (10⁻⁸-3·10⁻⁴ mol/L), an irreversible TG2 inhibitor that has been shown to lock the enzyme in the open conformation in crystallization studies, does not induce relaxation at any tested doses.

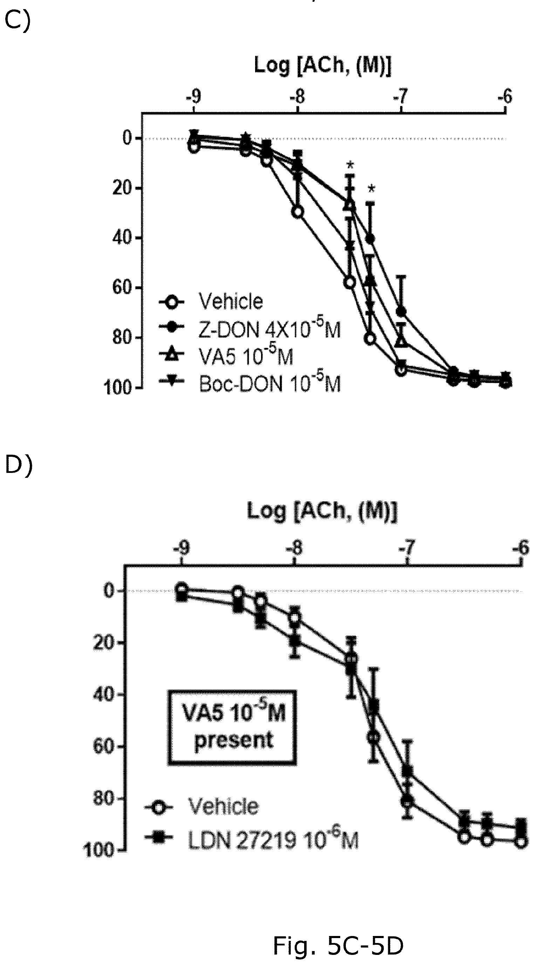

**Figure 5 C)** Shows that, in contrast to LDN-27219, membrane permeable inhibitors that force TG2 into the open conformation (Z-DON (4·10⁻⁵ mol/L) and VA-5 (10⁻⁵ mol/L)) reduce the endothelium-derived relaxation of mesenteric arteries extracted from ≈12 week Male Wistar rats when induced by acetylcholine (10⁻⁸-10⁻⁵ mol/L) and compared to the control experiments (vehicle) and membrane impermeable TG2 inhibitor Boc-DON-Gln-Ile-Val-OMe (from now on Boc-DON) (10⁻⁵ mol/L).
**Figure 5 D)** Shows that the presence of VA-5 (10⁻⁵ mol/L) prevents LDN-27219 (10⁻⁶ mol/L) to produce the improvement in endothelium-derived relaxation of small mesenteric arteries extracted from ≈12 week Male Wistar rats. This experiment was performed under the same conditions as the experiment shown in Figure 1 B, except from the presence of the irreversible TG2 inhibitor VA-5 that lock the enzyme in the open conformation.

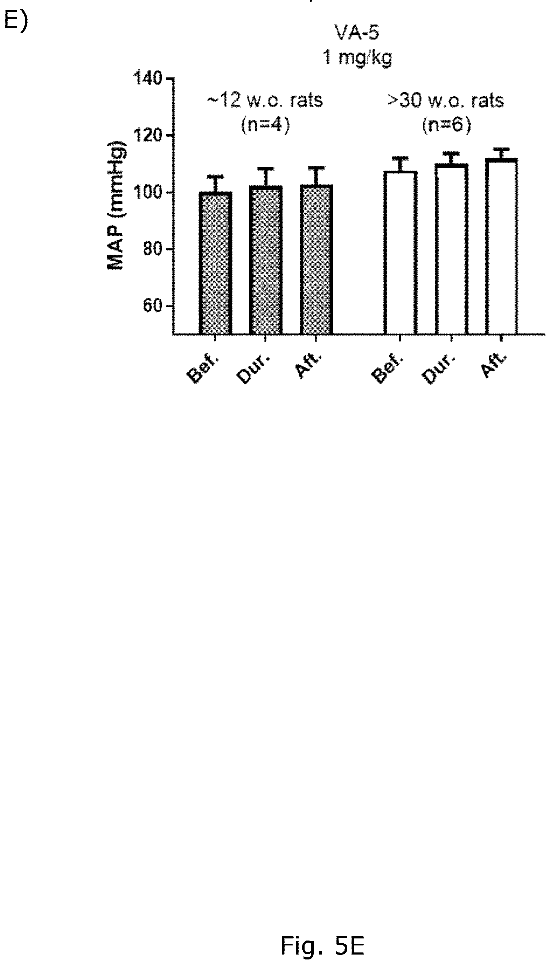

**Figure 5 E)** Shows the average mean blood pressure (mmHg) recorded before, during and after the infusion of a single dose of VA-5 (1 mg/kg) and illustrates the lack of anti-hypertensive effect of this compound in both younger rats (12-14 weeks old) and older rats (>30 weeks old).

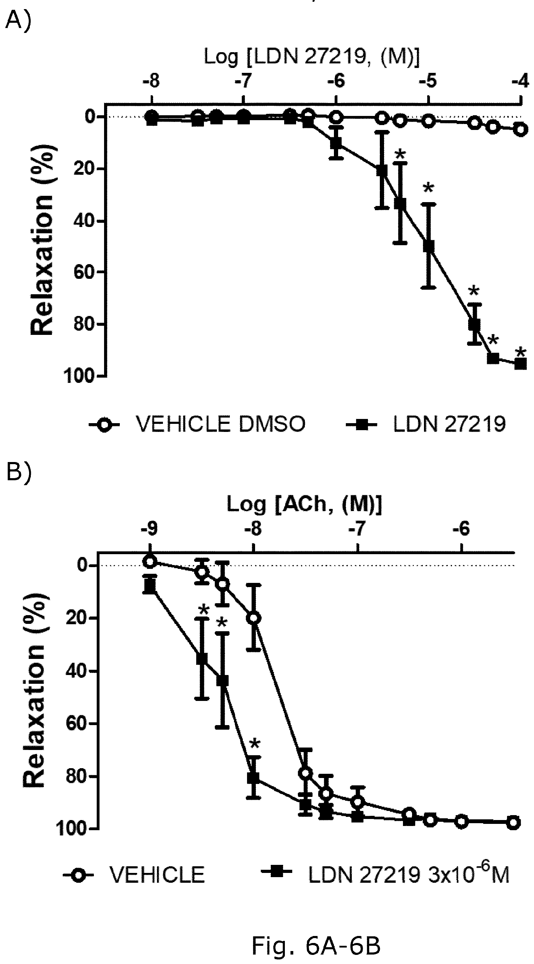

**Figure 6 A)** Shows that LDN-27219 (10⁻⁸-10⁻⁵ mol/L), induces a direct and dose-dependent relaxation of small subcutaneous arteries obtained from human biopsies. These results evidence the translatability to human tissue of the previous results in tissue from rodents (Figure 5B).
**Figure 6 B)** Shows that LDN-27219 (3·10⁻⁶ mol/L) improves the endothelium-derived relaxation of small subcutaneous arteries obtained from human biopsies as induced by acetylcholine (10⁻⁸-10⁻⁵ mol/L) as compared to the control experiments (vehicle). These results evidence the translatability to human tissue of the previous results in tissue from rodents (Figure 1).

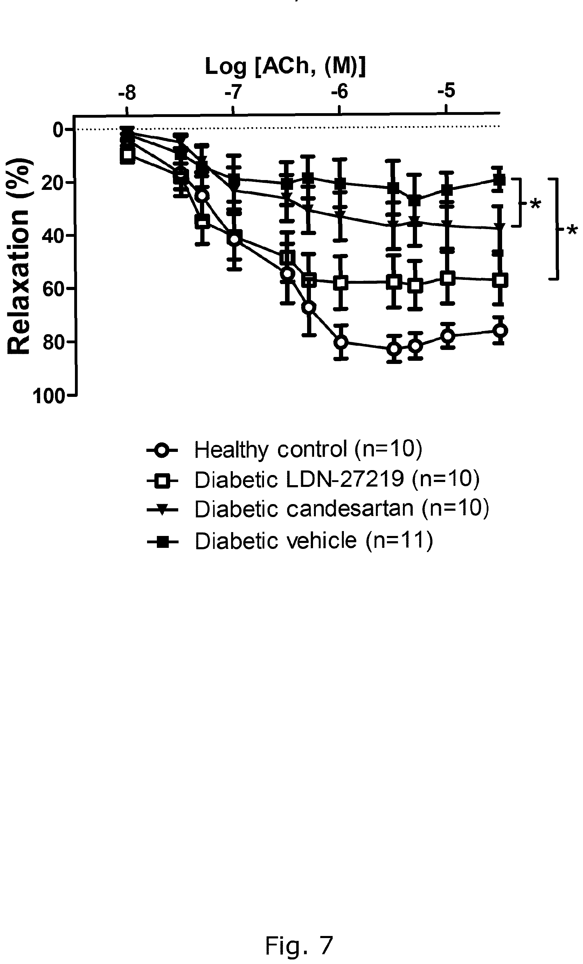

**Figure 7****)** Shows that intraperitoneal injection of LDN-27219 (8 mg/kg/12h) for 3 weeks (long-term *'in vivo'* study) is able to successfully prevent endothelial dysfunction in diabetic BKS mice of 10 weeks of age more effectively than oral candesartan (3 mg/kg/24 h), an anti-hypertensive treatment used in the clinical practice that has previously demonstrated to improve endothelial function in similar disease models of diabetes. As in previous figures, endothelial function is studied as relaxation of small mesenteric arteries as induced by acetylcholine (10⁻⁸-3·10⁻⁵ mol/L).

The present invention will now be described in more detail in the following.

### Detailed description of the invention

### Definitions

Prior to discussing the present invention in further details, the following terms and conventions will first be defined:

### Enzyme modulator

In the present context, an "enzyme modulator" is a compound that changes the activity of an enzyme. The modulator may be an enzyme inhibitor that decreases the activity of the enzyme, or an enzyme inducer that increases the activity of the enzyme. Specifically a "modulator of tissue transglutaminase" (TG2) may for example induce the formation of a closed conformation of TG2, and therefore inhibit the open conformation of TG2. In the present invention, the enzyme modulator may be a small organic molecule, such as a compound of Formula (I), more specifically the compound of Formula (II) [LDN-27219], which changes the conformation of the enzyme and thereby alters the activity of the enzyme.

### Tissue Transglutaminate (TG2)

In the present context, "Tissue Transglutaminase" (TG2) is an enzyme of the transglutaminase family (comprising TG1-TG7 and Factor XIII). The TG2 enzyme is highly expressed in endothelial and smooth muscle cells and can be found within many different organs of the body, including the brain, the heart, the liver and small intestine. TG2 is located in different intracellular and extracellular compartments and has several functions depending on the location and conformation of the enzyme. The intracellular TG2 is most abundant in the cytosol and in smaller amounts within the nucleus and mitochondria. TG2 is found in the body in a closed conformation and in an open confirmation. When TG2 is bound to Ca²⁺, it conforms to an open conformation that shows extracellular transamidase activity and leads to covalent cross-linking of structural proteins. In the closed conformation, it presents intracellular GTP-binding activity that enables it to act as a G protein (Gho) and consequently allow for signal transduction in the cell.

### Endothelial dysfunction

In the present context, "endothelial dysfunction" is a condition in which the endothelial layer, that coats the luminal surface of the vascular system, fails to function normally. Such a condition is associated with a decreased ability of the endothelial layer to secrete important mediators for control of vasoconstriction and vasodilation. Vasoconstriction is mostly regulated by endothelin-1 and thromboxane A2 mediators, whereas vasodilation is regulated by nitric oxide (NO), prostacyclin or other endothelium-derived hyperpolarization factors. The endothelial cells play an essential role in the regulation of vascular tone in response to hemodynamics and chemical signals. Thus, endothelial dysfunction is e.g. associated with increased blood pressure and cardiovascular diseases in the vascular system. Endothelial dysfunction particularly occurs in association with aging and diabetes. Dysfunction of the endothelial cells may also occur when excessive amounts of low-density lipoprotein (LDL) particles are oxidized within the endothelial layer. Although endothelial dysfunction may be regarded as a cardiovascular risk factor in itself, it is associated to a number of related pathologies/diseases such as for example dementia, Alzheimer's disease, ventricular hypertrophy, vascular calcification, atherosclerosis, ischemic chronic wounds, hypertension, and erectile dysfunction.

### Diabetes

In the present context, the term "diabetes" refers to the disease diabetes mellitus. It is a type of disease in which the amount of sugar in the blood is far removed from normal levels for a prolonged time period. In most cases the blood will contain an elevated amount of sugar, either because of insufficient production of insulin (diabetes 1) or because the cells do not respond properly to the insulin (diabetes 2). The abnormal levels of sugar in the blood stream cause a decrease in NO production by the endothelial cells and decreased activity of NO. Diabetes 1 is classified as an immune-mediated or idiopathic disease that is characterized by loss of the insulin-producing beta-cells in the pancreatic islets. Diabetes 1 covers roughly 10% of the present 422 million diabetes patients worldwide. Diabetes 2 is characterized by insulin resistance in the cells and is primarily caused by obesity instigated by lifestyle factors, such as lack of physical activity, poor diet, and stress. Diabetes 2 covers roughly 90% of the present 422 million diabetes patients worldwide.

### Age-associated endothelial dysfunction

In the present context, the term "age-associated endothelial dysfunction" refers to endothelium dysfunction in which the cells do not secrete the required amount of mediators controlling vascular tone as a consequence of an aging body. It also covers endothelium dysfunctions caused by elevated levels of cholesterol resulting from increasing age. Further, any diseases resulting from endothelial dysfunction may also be age-associated.

### Diabetes-associated endothelial dysfunction

In the present context, the term "diabetes-associated endothelial dysfunction" refers to endothelial dysfunction caused by diabetes and the resulting impairment of the NO production and activity of nitric oxide. Further, any diseases resulting from endothelial dysfunction may also be diabetes-associated.

### Optionally substituted

In the present context, the term "optionally substituted" describes any moiety that is obtained if one or more of the hydrogen atoms in any position on the optionally substituted moiety (such as e.g. an aromatic ring) is substituted with another atom or group.

### Heteroaryl

In the present context, the term "heteroaryl" refers to a compound comprising one or more aromatic rings that consist of carbon atoms and at least one or more heteroatoms, such as O, N or S. Examples of 5, 6, 9 and 10 membered heteroaryls include pyrrole, furane, thiophene, thiazole, isothiazole, oxazole, isooxazole, pyrazole, imidazole, 1,2,3-oxadiazole, 1,2,4-oxadiazole, 1,2,5-oxadiazole, 1,3,4-oxadiazole, 1,2,3-triazole, 1,2,4-triazole, pyridine, pyridazine, pyrimidine, pyrazine, 1,2,4-triazine, 1,3,5-triazine, indole, indolizine, indazole, benzimidazole, 4-azaindole, 5-azaindole, 6-azaindole, 7-azaindole, 7-azaindazole, pyrazolo[1,5-a]pyrimidine, benzofuran, isobenzofuran, benzo[b]thiophene, benzo[c]thiophene, benzo[d]isoxazole, benzo[c]isoxazole, benzo[d]oxazole, benzo[c]isothiazole, benzo[d]thiazole, benzo[c][1,2,5]thiaciazole, 1H-benzotriazole, quinolone, isoquinoline, quinoxaline, phthalazine, quinazoline, cinnoline, 1,8-naphthyridine, pyrido[3,2-d]pyrimidine, pyrido[4,3-d]pyrimidine, pyrido[3,4-b]pyrazine, pyrido[2,3-b]pyrazine.

### Alkyl

In the present context, the term "alkyl" means any terminal saturated hydrocarbon chain, including branched or linear chains. Examples include methyl (-CH₃), ethyl (-CH₂CH₃), propyl (-CH₂CH₂CH₃) and isobutyl (-CH(CH₃)CH₂CH₃). C₁-C₁₀ alkyl refers to an alkyl comprising 1-10 carbons.

### Alkenyl

In the present context, the term "alkenyl" refers to an alkyl as described above, which however is unsaturated and contains at least one or more double bonds within its structure.

### Alkynyl

In the present context, the term "alkynyl" refers to an alkyl as described above, which however is unsaturated and contains at least one or more triple bonds within its structure.

### Pharmaceutically acceptable

In the present context, the term "pharmaceutically acceptable" refers to molecular entities and compositions that are physiologically tolerable and do not typically produce an allergic or similar untoward reaction, such as e.g. gastric upset, dizziness and the like, when administered to a human. Preferably, as used herein, the term "pharmaceutically acceptable" means approved by a regulatory agency of the Federal or a state government or listed in the U.S. Pharmacopoeia or other generally recognized pharmacopoeia for use in animals, and more particularly in humans.

### Solution

In the present context, the term "solution" refers to a homogeneous mixture composed of two or more substances.

### Solid dosage form

In the present context, the term "solid dosage form" refers to a solid pharmaceutical product consisting of a defined mixture of pharmaceutical active ingredients and inactive components. When apportioned into a specific dose, a solid dosage form may be termed a solid unit dosage form. Solid dosage formulations may be in the form of tablets, capsules, granules, powders, sachets, or chewables.

### An effective amount

In the present context, the term "effective amount" refers to a dosage sufficient to produce a desired result on a health condition, pathology, and disease of a subject or for a diagnostic purpose. The desired result may comprise a subjective or objective improvement in the recipient of the dosage. "Therapeutically effective amount" refers to that amount of an agent effective to produce the intended beneficial effect on health.

The present invention relates to the treatment of endothelial dysfunction via selective modulation of the tissue transglutaminase (TG2) enzyme using compounds as defined in claim 1 that stabilize the enzyme in the so-called "closed conformation", where GTP binds to the enzyme. As provided above this leads to enhanced endothelial function and a potential new pathway for treating diseases such as for example hypertension, erectile dysfunction and others.

Thus a first aspect of the present invention is a compound which is a modulator of tissue transglutaminase (TG2) as defined in claim 1 for use in the treatment of endothelial dysfunction.

In one embodiment the modulator of tissue transglutaminase (TG2) as defined in claim 1 is for use in the treatment of diseases resulting from endothelial dysfunction.

The present inventors have shown that compounds promoting the closed conformation of TG2 and thus inhibiting or lowering the available amount of the open conformation of TG2 are particularly effective and selective in improving endothelial function. Therefore, the modulator of tissue transglutaminase (TG2) as defined in claim 1 may preferably be a promoter of the closed conformation of tissue transglutaminase, and even more preferably the modulator of tissue transglutaminase (TG2) may be a promoter of the closed conformation of tissue transglutaminase and an inhibitor of the open conformation of tissue transglutaminase. Preferably, the tissue transglutaminase (TG2) is human tissue transglutaminase (EC 2.3.2.13).

Age and diabetes may lead to increased expression of TG2. In a particularly preferred embodiment the modulator of tissue transglutaminase (TG2) as defined in claim 1 has an effect on endothelial function which is age-dependent. Particularly preferred are compounds which increasingly improve endothelial function with increasing age of a subject, to which the compound is administered. Likewise, compounds which improve endothelial function in particularly in diabetic subjects are preferred.

As described a number of small molecule inhibitors of TG1-7 are known including inhibitors of TG2, but the majority are general inhibitors/modulators of TG2 (i.e. inhibits both the closed and open form). Since promotion of the closed form over the open form is hypothesised to have beneficial effects in endothelial function, compounds achieving such modulation would have potential in the treatment of endothelial dysfunction. The present inventors have found that LDN-27219 and derivatives achieve such promotion of the closed conformation while inhibiting the open conformation. These compounds are superior to non-specific inhibitors and inhibitors that lock the enzyme in the open conformation in terms of treating endothelial dysfunction, particularly in patient sub-groups of old age and sub-groups diagnosed with diabetes. Suitable derivatives of LDN-27219 are described in e.g. US 2006/0183759.

Hence, according to the invention the modulator of tissue transglutaminase (TG2) is a compound of formula (I): 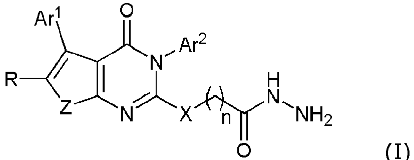 wherein
- Z is selected from the group consisting of S, SO₂, and Se,
- Ar¹ and Ar² are independently selected from the group consisting of optionally substituted phenyl and optionally substituted heteroaryl,
- R is selected from the group consisting of hydrogen, deuterium, halogen, and C₁-C₃ alkyl,
- X is selected from the group consisting of O, NH, N(Me), and S,
- n is an integer selected from the group consisting of 1, 2 and 3.

In the present context, SO₂ is a diradical having the formula 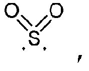 which bond to other atoms in a molecular structure through covalent bonds to the S in SO₂.

In a more preferred embodiment Z is S.

The optional substituents of Ar¹ and Ar² may be selected from the group consisting of C₁-C₁₀ alkyl, C₂-C₁₀ alkenyl, C₂-C₁₀ alkynyl, amino (-NH2), - CH₂NH(C₁-C₁₀ alkyl), -CH₂N(C₁-C₁₀ alkyl)2, aminoalkyl (-NH(C₁-C₁₀ alkyl) or - N(C₁-C₁₀ alkyl)₂, cyano (-CN), -CONH₂, -CONH(C₁-C₁₀ alkyl), -CON(C₁-C₁₀ alkyl)₂, hydroxyl (-OH), C₁-C₁₀ alkyl hydroxyl (-alkyl-OH), C₁-C₁₀ alkoxy(-O-alkyl), carboxylic acid (-COOH), C₁-C₁₀ alkyl esters (-COO-alkyl), C₁-C₁₀ alkyl acyl (-CO-alkyl), sulfonic acid (-SO₃H), C₁-C₁₀ alkyl sulfonate (-SOs-alkyl), phosphonic acid (-PO(OH)₂), C₁-C₁₀ alkyl phosphonate (-PO(O-alkyl)₂), phosphinic acid (-P(O)(H)OH), SOzNHz, hydroxamic acid (-CONHOH), C₁-C₁₀alkyl sulfonylureas (-NHCONHSO₂(alkyl)), C₁-C₁₀ acylsulfonamides (-SO₂-NHCO-(alkyl), hydroxyl amine (-NHOH), nitro (-NO₂), and halogen.

More preferably the optional substituents of Ar¹ and Ar² are selected from the group consisting of C₁-C₁₀ alkyl, cyano (-CN), -CONH₂, hydroxyl (-OH), C₁-C₁₀ alkyl hydroxyl (-alkyl-OH), carboxylic acid (-COOH), C₁-C₁₀ alkyl esters (-COO-alkyl), sulfonic acid (-SO₃H), phosphonic acid (-PO(OH)₂), nitro (-NO₂), and halogen.

Even more preferably the optional substituents of Ar¹ and Ar² are selected from the group consisting of C₁-C₃ alkyl, hydroxyl (-OH), C₁-C₃ alkyl hydroxyl (-alkyl-OH), and halogens. Halogens may preferably be selected from the group consisting of fluorine, chlorine, bromine, and iodine, preferably fluorine.

In a preferred embodiment the compound according to Formula (I), is a compound wherein
- Ar¹ and Ar² are optionally substituted phenyl,
- R is hydrogen or halogen,
- X is S,
- n is an integer selected from the group consisting of 1, 2 and 3.

In an even more preferred embodiment the compound according to Formula (I), is a compound wherein
- Ar¹ and Ar² are phenyl,
- R is hydrogen,
- X is S,
- n is 1.

Thus, in a most preferred embodiment the modulator of tissue transglutaminase (TG2) is a compound of formula (II):

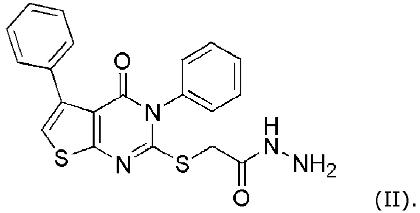

The compound of formula (II) corresponds to LDN-27219, and hence, in an alternative embodiment the modulator of tissue transglutaminase (TG2) is LDN-27219.

As described above for the present first aspect of the invention, it relates to the treatment of endothelial dysfunction, and diseases resulting therefrom. The present inventors have found that the selective TG2 modulators of the invention are increasingly effective in the treatment of endothelial dysfunction the older the subjects are. This may be related to the fact that TG2 is increasingly expressed in aging subjects.

Therefore, in a preferred embodiment of the present invention the endothelial dysfunction is associated with increased levels or increased activity of tissue transglutaminase (TG2). More preferably the endothelial dysfunction is age-associated, i.e. associated with changes in endothelial function related to aging.

In terms of age-associated endothelial dysfunction and age-associated diseases resulting from endothelial dysfunction, a patient sub-group may be defined, wherein the compounds of the present invention have pronounced beneficial effects. Thus, in a preferred embodiment the compounds of the invention are administered to human patients between the age of 30-140 years, such as the age of 35-140 years, 40-140 years, 45-140 years, 50-140 years, 55-140 years, 60-140 years, 65-140 years, 70-140 years, such as preferably 30-120 years, 40-100 years, such as most preferably 50-100 years.

Expression of TG2 is also increased in diabetic subjects. The inventors have found that the selective TG2 modulators for the closed conformation in the present invention are especially effective in the treatment of endothelial dysfunction associated with diabetes i.e. associated with changes in endothelial function related to diabetes.

Thus, in a preferred embodiment the endothelial dysfunction is diabetes-associated. Thus, a preferred sub-group of patient may be defined as patients or subjects suffering from diabetes or diagnosed with diabetes, also known as diabetes mellitus. Diabetes may include diabetes-1 or diabetes-2. Thus, in a more preferred embodiment of the present invention the endothelial dysfunction is associated with diabetes-1 or diabetes-2, preferably diabetes-2.

Age and diabetes may often be related, particularly diabetes-2 may develop in aging patient. Hence, in another preferred embodiment of the present invention the endothelial dysfunction is both age-associated and diabetes-associated, particularly it may be both age-associated and diabetes-2 associated.

The subjects or patients treated for the present invention are preferably mammals, such as preferably human subjects or patients.

A number of diseases are known to be a direct or indirect result of endothelial dysfunction, or to be associated with endothelial dysfunction. Such related diseases comprise cardiovascular diseases, diseases related to vasodilative function, but also diseases related to the brain and its function. Also, the well-known antifibrotic effects of TG2 inhibitors, together with the vasoprotective effects observed from the induction of the closed conformation, make LDN-27219 potentially useful in the treatment of fibrotic diseases. Hence, in a preferred embodiment of the present invention the disease resulting from endothelial dysfunction is selected from the group consisting of dementia, Alzheimer's disease, ventricular hypertrophy, vascular calcification, renal fibrosis, cardiac fibrosis, pulmonary fibrosis, atherosclerosis, ischemic chronic wounds, hypertension, pulmonary hypertension, and erectile dysfunction, preferably hypertension and erectile dysfunction.

For treatment of erectile dysfunction, it is possible to define a patient sub-group, in which the compounds of the present invention have a pronounced effect. Thus in the treatment of erectile dysfunction the compounds may preferably be administered to patients of male gender.

In an embodiment of the present invention the compound of the present invention is administered in a composition further comprising a pharmaceutically acceptable carrier or diluent, more preferably in the form of a solution or a solid dosage form, and most preferably as a solid dosage form. In an embodiment of the present invention the solid dosage form is selected from the group consisting of a tablet, a capsule, and a sachet.

The compounds of the present invention and the composition as described above may be administered orally, or parenterally, such as intravenously, intradermally, subcutaneously, or topically. Preferably the compound is administered in a therapeutically effective amount.

One particularly preferred embodiment of the invention is a compound of formula (I): 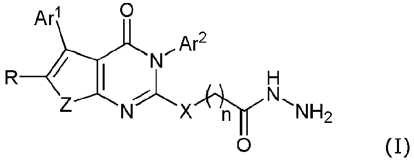 wherein
- Z is selected from the group consisting of S, SO₂, and Se,
- Ar¹ and Ar² are independently selected from the group consisting of optionally substituted phenyl and optionally substituted heteroaryl,
- R is selected from the group consisting of hydrogen, deuterium, halogen, and C₁-C₃ alkyl,
- X is selected from the group consisting of O, NH, N(Me), and S,
- n is an integer selected from the group consisting of 1, 2 and 3,
for use in the treatment of a disease selected from the group consisting of dementia, Alzheimer's disease, ventricular hypertrophy, vascular calcification, atherosclerosis, ischemic chronic wounds, hypertension, and erectile dysfunction.

Another particularly preferred embodiment of the invention is a compound of formula (I): 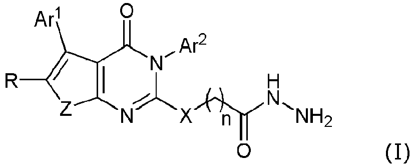 wherein
- Z is selected from the group consisting of S, SO₂, and Se,
- Ar¹ and Ar² are independently selected from the group consisting of optionally substituted phenyl and optionally substituted heteroaryl,
- R is selected from the group consisting of hydrogen, deuterium, halogen, and C₁-C₃ alkyl,
- X is selected from the group consisting of O, NH, N(Me), and S,
- n is an integer selected from the group consisting of 1, 2 and 3,
for use in the treatment of endothelial dysfunction,
wherein said endothelial dysfunction is age-associated or diabetes-associated.

Yet another particularly preferred embodiment of the invention is a compound of formula (I): 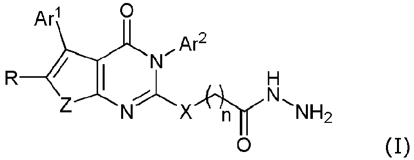 wherein
- Z is selected from the group consisting of S, SO₂, and Se,
- Ar¹ and Ar² are independently selected from the group consisting of optionally substituted phenyl and optionally substituted heteroaryl,
- R is selected from the group consisting of hydrogen, deuterium, halogen, and C₁-C₃ alkyl,
- X is selected from the group consisting of O, NH, N(Me), and S,
- n is an integer selected from the group consisting of 1, 2 and 3,
for use in the treatment of diseases resulting from endothelial dysfunction, wherein the compound of formula (I) is administered to human patients between the age of 30-140 years.

Yet another particularly preferred embodiment of the invention is a compound of formula (I): 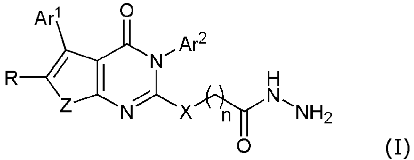 wherein
- Z is selected from the group consisting of S, SO₂, and Se,
- Ar¹ and Ar² are independently selected from the group consisting of optionally substituted phenyl and optionally substituted heteroaryl,
- R is selected from the group consisting of hydrogen, deuterium, halogen, and C₁-C₃ alkyl,
- X is selected from the group consisting of O, NH, N(Me), and S,
- n is an integer selected from the group consisting of 1, 2 and 3,
for use in the treatment of diseases resulting from endothelial dysfunction, wherein the compound of formula (I) is administered to human patients diagnosed with diabetes.

A second aspect of the present invention is a compound of formula (I) for use in a method of treating endothelial dysfunction comprising administering to a subject in need thereof a compound of formula (I) which is a modulator of tissue transglutaminase (TG2).

It should be noted that embodiments and features described in the context of one of the aspects of the present invention also apply to the other aspects of the invention, in as far as covered by the claims.

The invention will now be described in further details in the following non-limiting examples.

### Examples

### Materials and methods

For the conformational shift assay using native polyacrylamide gel electrophoresis (native-PAGE) purified and functional human TG2 protein produced in insect cells was obtained from Zedira (Darmstadt, Germany).

The following compounds were used: ACh, Candesartan cilexetil, Na₂GTP (guanosine 5'-triphosphate sodium salt), phenylephrine, and U46619 (9α-epoxymethanoprostaglandin F2α) from Merk (Darmstadt, Germany). LDN-27219 was obtained from Tocris Bioscience (Bristol, UK). Boc-DON and Z-DON were obtained from Zedira (Darmstadt, Germany). Sulfobutyl ether beta-cyclodextrin sodium (SBE-b-CD) was obtained from Glentham Life Sciences (Wiltshire, United Kingdom). VA-5 was from ChemShuttle (Hayward, CA, USA). Synthesis of LDN-27219 is described in US 2006/0183759.

Unless otherwise stated all substances were dissolve in distilled water. For'ex *vivo'* experiments U46619 was dissolved in ethanol and Boc-DON, LDN-27219 VA-5 and Z-DON were dissolved in DMSO and stored at -20°C. Further dilution of the above mentioned compounds were made in distilled water. The DMSO concentration in the bath were kept below % and did not affect vessel 0.01 contractility. For *'in vivo'* experiments LDN-27219 and VA-5 were dissolved in PEG-400.

For long-term *'in vivo'* experiments, stocks of LDN-27219 dissolved in PEG-400 were diluted in saline solution containing SBE-b-CD to improve solubility (9 mg/mL NaCl; 15 % (w/v) SBE-b-CD) immediately after injection, resulting in a final concentration of 2.00 mg/mL of LDN-27219 in 10% PEG-400/90%(15% (w/v) SBE-b-CD in saline solution). For the treatment on the drinking water 1 mg/ml Candesartan cilexetil was dissolved in a vehicle of polyethylene glycol 400 (10% v/v), ethanol (5% v/v), cremophor EL (2% v/v), and tap water (83% v/v) and the pH was adjusted to 9.0 with 0.5 mol/L NazCOs prior to dilution to the final concentration in tap water, as previously described (Seltzer A., et al., Brain Res., 2004, 1028(1), 9-18). Final concentrations needed to achieve the desired dosis of candesartan cilexetil per day were determined by measuring water consumption each 3-4 days.

### Animals and tissue preparation

Human subcutaneous arteries were obtained from fat biopsies of the gluteal region from both male and female patients (30-70 years of age) with essential hypertension.

Male Wistar rats of 6-7, 12-14 and 30-35 weeks of age, weighing 200-250, 300-400 and 600-700 g respectively, were obtained from Taconic Denmark ApS (Rye, Denmark) and housed adequately in the animal facility of Aarhus University. Healthy male BKS(D)-Leprdb/JOrlRj heterozygous mice of age 14-16, 24-25 (for '*ex vivo'* studies) and 6-7 weeks (for long-term *'in vivo'* studies) and diabetic homozygous mice of age 16-17 (for '*ex vivo'* studies) and 6-7 (for long-term *'in vivo'* studies) weeks were obtained from Taconic, Ry, Denmark or Janvier Labs (Le Genest-Saint-Isle, France) and housed adequately in the animal facility of Aarhus University.

For '*ex vivo'* studies, animals were randomly selected and euthanized by cervical dislocation and exanguination. The mesenteric arteries were immediately extracted and placed in 4°C physiological saline solution (PSS, pH= 7.4) of the following composition (mmol/L): 119 NaCl, 4.7 KCl, 1.18 KH₂PO₄, 1.17 MgSO₄, 1.5 CaCl₂, 24.9 NaHCOs, 0.026 EDTA and 5.5 glucose and small mesenteric arteries (internal diameters of 200-300 µm) were isolated. Human subcutaneous arteries (internal diameters of 300-400 µm) were dissected from the biopsies in the same conditions mentioned above for the mesenteric bed from rodents.

For *'in vivo'* experiments Wistar rats of different age were fasted 8 h before anesthesia with 35 mg/kg of S-ketamine and 50 mg/kg of pentobarbital sodium administered intraperitoneally.

For long-term *'in vivo'* studies, diabetic homozygous mice were randomly allocated into three groups: 1) treatment with Intraperitoneal (IP) injections of LDN-27219 (8 mg/kg/12h) and vehicle drinking water [LDN-27219 treatment group], or 2) treatment with IP injections of vehicle (each 12 h) and candesartan cilexetil (3 mg/kg/24h) in the drinking water [candesartan treatment group], or 3) treatment with IP injections of vehicle (each 12 h) and vehicle drinking water [vehicle treatment group]. Age-paired healthy heterozygous animals were included as healthy controls, receiving the same treatment as the vehicle treatment group. After three weeks of treatment, animals were anesthetized with inhalation of isofluorane and euthanized by cervical dislocation. Small mesenteric arteries were obtained as described above.

Animal protocols and care were approved by the Danish Animal Experiments Inspectorate (permission 2014-15-2934-0159) and followed the ARRIVE guidelines (McGrath et al, 2015). The study using human arterial tissue was approved by the Regional Ethics Committee, Central Denmark (permission number: 1-10-72-120-17) and conducted in accordance with the principles of the Helsinki Declaration II for medical research. All participants gave informed consent prior to participation.

### Example 1 - Effects of LDN-27219 in rats of different age (ex-vivo)

Small mesenteric arteries were mounted in microvascular myographs (Danish Myotechnology, Aarhus, Denmark) using two 40 µm wires and stretched to their optimal diameter for isometric tension recordings, which corresponded to 0.9 times the estimated internal diameter at 100 mmHg of transmural pressure. PSS solution in the myograph bath was heated to 37°C and continuously bubbled with 5% CO2 in air to maintain pH (7.4). The vessel viability was tested at the beginning of each experiment by measuring the vasoconstrictor responses to a solution with a high K⁺ concentration (KPSS), equivalent to PSS except that NaCl was exchanged for KCl on an equimolar basis, giving a final concentration of 123,7 mmol/L K⁺ and the presence of endothelium was confirmed by addition of acetylcholine (ACh) (10⁻⁵) after pre-constriction with noradrenaline (5·10⁻⁶ mol/L), arteries with less than 75% of relaxation were discarded.

Mesenteric arteries from rats of different age were incubated with (10⁻⁶ mol/L) LDN-27219 or vehicle for 25 minutes and pre-contracted with phenylephrine (Phe) (10⁻⁵ mol/L). Then cumulative addition of acetylcholine (ACh) caused the relaxation of the mesenteric arteries and the concentration-response curves were constructed.

Rats of 5-7 weeks age, about 12 weeks age, and more than 30 weeks of age were tested respectively. The results of the isometric tension studies are shown in Figure 1, A), B) and C),respectively, each representing a different, age group.

Example 1 demonstrates that LDN-27219 allows a more potent endothelium-dependent relaxation of the mesenteric arteries when compared to the ones treated with vehicle, and that the effect of LDN-27219 is more pronounced in old rats, than in young rats.

### Example 2 - Effects of LDN-27219 in diabetic mice and healthy control mice of different age (ex-vivo)

In these experiments, healthy control mice of different ages and diabetic adult mice were used and the relaxation measurements were performed as described in Example 1.

Healthy male BKS(D)-Leprdb/JOrlRj heterozygous mice between 14-16 and 24-25 weeks of age, and diabetic homozygous mice between 16-17 weeks of age were tested respectively. The results of the isometric tension studies are shown in Figure 2, A), B) and C),respectively.

Example 2 demonstrates that LDN-27219 allows a more potent endothelium-dependent relaxation of the mesenteric arteries when compared to the vehicle experiments in healthy and diabetic mice, and that the effect of LDN-27219 is more pronounced in older mice, and particularly pronounced in diabetic mice.

### Example 3 - Effects of LDN-27219 rats of different age (in vivo)

Mean arterial pressure (MAP) *'in vivo'* measurements:
Male Wistar rats with 12-14 and 30-35 weeks of age were anesthetized with s-ketamine (35 mg/kg) and pentobarbital (50 mg/kg) and placed on a heated blanket to maintain body temperature at 37°C. ECG needles were installed for electrocardiographic measurements and a solid state catheter (Millar Inc, Houston, USA) was introduced into the carotid artery to measure MAP. MAP and electrocardiographic data was continuously recorded on a computerized data acquisition system (PowerLab, ADInstruments). Adequate depth of anesthesia was checked periodically by the absence of the toe pinch withdrawal effect and 1/3 of the initial dose of anesthetics was administered intraperitoneally when needed (usually each 1½ h). Changes in MAP and electrocardiographic signal in response to intrajugular infusion during 3 minutes of different doses of LDN-27219 and the corresponding vehicle (PEG-400) were studied.

The Area Under the Curve (AUC) was calculated using Graphpad Prism 5.1 (GraphPad Software, San Diego, California, USA) for each response to the change in MAP that was collected during the experiments in example 3. The AUC gives information about the maximal response, and about the duration of the effect.

The mean arterial pressure (MAP) over 3 minutes when adding LDN-27219 and control is shown in Figure 3 A), whereas the Area Under the Curve for these MAP measurements are shown for different aged rats in Figure 3 B) as a function of LDN-27219 concentration.

Preliminar '*in vivo*' studies in healthy male BKS(D)-Leprdb/JOrlRj heterozygous mice showed that, additionally to the previously described antyhipertensive effect, mice displayed increased erectile function, measured as peak intracavernosal pressure (PICP) over MAP, upon the administration of LDN-27219.

Example 3 demonstrates that LDN-27219 has an antihypertensive effect *in vivo,* as it lowers the mean blood pressure in the carotid artery. Example 3 further demonstrates the greater effect of LDN-27219 in older animals, which is consistent with the results obtained in Example 1.

### Example 4 - LDN-27219 promotes closed confirmation of TG2 as compared to TG2 inhibitors that lock the enzyme in the open conformation.

*LDN-27219 is able to increase the percentage of TG2 present in the close conformation 'in vitro' compared to vehicle.*

Native gel electrophoresis experiments were performed using a method similar to those previously described (Pinkas DM, et al., PLoS Biol, 2007, 5(12), 2788-2796.) (Iversen R, et al., Proc Natl. Acad. Sci., 2014, 111(43), 17146-17151). Briefly, TG2 (2.5 uM) was incubated for 30 min at room temperature in preincubation buffer (75 mM imidazole, 0.5 mM EDTA, 5 mM DTT [pH 7.2], 10% DMSO) with or without 1 mM GTPNa₂ or 1 mM LDN-27219 before adding Native Sample Buffer for Protein Gels (Bio-Rad) or in preincubation buffer with 0.2 mM Z-DON 30 min prior addition of 5mM of CaCl₂ for 20 min. Native buffer was added (dilution 1:2), and 1.5 ug of protein was loaded onto a 4-20% Criterion^{™} TGX Stain-Free^{™} gel (Bio-Rad) using Tris-glycine as the running buffer. For 75 min, 125 V was applied at 4 °C. The bands were visualized using stain-free technology by exposing the gels to UV light and capturing the image using a PXi 4 Touch image analysis system (Syngene). Band intensity analysis was carried out using GeneTools 4 software (Syngene).

Figure 4 A) and B) show that LDN-27219 is able to increase the relative abundance of the closed conformation, reducing therefore the relative abundance of the open conformation, compared to vehicle. While GTP is able to completely force TG2 to its closed conformation of the enzyme and, contrarily, Z-DON in presence of calcium is able to force its open conformation (Figure 4 C) and D)).

*At the cellular level, LDN-27219 has the same influence in vascular smooth muscle cells (VSMCs) potassium currents as an endogenous promoter of the closed conformation of TG2, while the induction of the open conformation of the enzyme has the opposite effect.*

For isolation of VSMCs, rat mesenteric arteries were dissected as previously described, opened longitudinally and stored in ice-cold dissociation solution containing (mmol/L): 119 NaCl, 4.7 KCl, 1.18 KH₂PO₄, 1.17 MgSO₄, 24.9 NaHCO₃, 0.027 EDTA, and 11 glucose, pH= 7.4. Arteries were then equilibrated during 10 min in a dissociation solution with bovine albumin serum (BSA; 1 mg/mL) at 37°C and then exposed to the same solution supplemented with papain (0.5 mg/mL; Worthington Biochemical Corp., Lakewood, NJ, USA) and dithiothreitol (DTT; 1 mg/mL) at 37°C for 8 min. Arteries were then washed in ice-cold dissociation solution and moved to dissociation solution containing BSA (1 mg/mL) and collagenase (0.7 mg/mL type F and 0.4 mg/mL type H; Worthington Biochemical Corp.) at 37°C for 3 min. After washing in ice-cold dissociation solution, isolated SMCs were obtained by gentle trituration of the digested arteries with a fire polished pipette into the SMC extracellular solution (for composition, see below). After the digestion protocol, SMCs were seeded in micro-dishes (ibidi GmbH, Martinsried, Germany) and used for patch clamp recordings 15 min after the isolation protocol and within the next 5 hours.

Extracellular patch-clamp solution for VSMCs contained the following (mmol/L): 130 NaCl, 5 KCl, 1.2 MgCl₂, 1.5 CaCl₂, 10 glucose and 10 HEPES (adjusted to pH 7.3 with NaOH) The patch pipette (intracellular) solution for SMCs contained the following (mmol/L): 130 KCl, 1.2 MgCl₂, 0.1 EGTA and 10 HEPES (adjusted to pH 7.2 with KOH). To study the effect of intracellular GTP in LDN-27219 activity over potassium currents, patch pipette solution with 5 mmol/L Na₂GTP was used in some experiments.

Membrane currents were recorded under the whole-cell configuration of the patch-clamp technique using an Axon Multiclamp 700A amplifier (Axon instruments, Molecular Devices, CA, USA). Patch pipettes were made of borosilicate glass capillaries (World Precision Instruments, FL, USA) fabricated using a dual-stage puller PP-830 (Narishige, Tokyo, Japan) and had resistances of 4-7 MΩ. Current recordings were digitized on line at 10 kHz and low pass filtered at 2 kHz using a Digidata 1440 A (Axon Instruments), to acquire and store data the pClamp 10 software was used. Current-voltage relations were determined using 600 ms voltage ramps from -100 to 160 mV and a holding potential (Vh) of -65 mV for SMCs.

Amplitudes of K⁺-outward currents were measured in control conditions and 12 min after the addition of LDN-27219 (3·10⁻⁵ mol/L) to the extracellular solution by a perfusion system. To study how conformational modulation of TG2 affected LDN-27219 activity over potassium channels, the initial protocol was repeated after one hour incubation with VA-5 (5·10⁻⁵ mol/L) and with GTP present in the intracellular solution, as VA-5 has been shown to force TG2 into the open conformation while GTP has been shown to induce the closed conformation.

Figure 5 A) shows that VSMCs treated with LDN-27219 had potassium currents significantly bigger compared to the ones in control conditions, being this currents equivalent to the ones recorded in presence of intracellular GTP. Contrarily, VSMCs treated with VA-5 presented significantly smaller potassium currents compared to those in control conditions. These results suggest that LDN-27219 mimics GTP, inducing the closed conformation of TG2 and leading to the opening of potassium channels, while VA-5 produces the opposite effect.

### Different mechanisms of TG2 inhibition on vascular tone

The small mesenteric arteries were prepared in the same way is described in example 1.

The small mesenteric arteries were pre-contracted with the thromboxane analogue, U46619 (4·10⁻⁷ mol/L) and concentration-response curves for TG2 specific inhibitors, LDN-27219 (10⁻⁸-10⁻⁵ mol/L) and Z-DON (10⁻⁸-3·10⁻⁴ mol/L) were constructed. To study the role of the endothelial pathways in LDN-27219 relaxation, concentration-response curves were constructed for it in arteries whose endothelium had been removed mechanically by rubbing the lumen repeatedly with a human scalp hair.

LDN-27219 induced relaxation in U46619 pre-contracted arteries as shown in Figure 5B). Furthermore, the results obtained in the same experiment using arteries without an endothelium layer showed a significantly reduced relaxation. Experiments were performed with cystamine in M. Engholm et al., British J. Pharm. (2016), 173, 839-855, showing that general TG2 inhibitors with unkown effects on TG2 conformation lacked effects on endothelial pathways. Also, the irreversible inhibitor Z-DON that has been shown to lock the enzyme in the open conformation, failed to induce any vasodilation. The results presented in Figure 5 B) are consistent with what is shown in Example 1 and indicate that the vasodilatory effects of LDN-27219 are associated with an endothelial pathway and that different mechanisms of TG2 inhibition have completely different effects in the vascular system.

*Pharmacological induction of TG2 closed conformation potentiates endothelial function while promotion of its open conformation reduces endothelium-dependent vasodilation.*

The small mesenteric arteries were prepared in the same way is described in example 1.

To investigate the effect of TG2 conformational modulation in endothelial function, arteries from animals of different age were pre-contracted with phenylephrine (Phe) (10⁻⁵ mol/L) and ACh concentration-response curves were constructed after a 1 hour incubation with the irreversible TG2 inhibitors Z-DON (4·10⁻⁵ mol/L), Boc-DON (10⁻⁵ mol/L) and VA-5 (10⁻⁵ mol/L), which have demonstrated to lock the enzyme in its open conformation.

Incubation with LDN-27219 during 25 min increased the response to ACh in Phe pre-constricted arteries from 12-14 weeks old rats as observed in Example 1 (Figure 1 B). Contrarily, in these experiments, incubation with Z-DON and VA-5, irreversible and membrane permeable TG2 inhibitors that lock the enzyme in its open conformation, caused a slight inhibition of ACh relaxation compared to the vehicle, while non-permeable TG2 inhibitor Boc-DON had no significant effect as shown in Figure 5 C). LDN-27219 potentiation of ACh-induced vasodilation was reduced or completely inhibited by the presence of these TG2 irreversible inhibitors as represented by VA-5 in Figure 5 D) These results indicate that the effects of LDN-27219 are mainly due to its capacity to induce the closed conformation of TG2.

*Inducing TG2 open conformation by pharmacological means has no effects in blood pressure 'in vivo'.*

Rats of different ages in these experiments were anesthetized, prepared and catheterized as described in Example 3. Intrajugular infusion during 3 minutes of a single dose (1 mg/kg) of the irreversible TG2 inhibitor VA-5, which has been shown to lock TG2 in the open conformation, did not produce any change in the mean blood pressure of the aorta neither in adult, nor in older animals. These results are shown in Figure 5 E) as average mean arterial pressure (MAP) values (mmHg) measured before (Bef.), during (Dur.) and after (Aft.) the infusion. These results indicate, consistently with the *ex-vivo* results, that inhibitors that lock the enzyme in the open conformation lack the antihypertensive properties of LDN-27219.

This example demonstrates that LDN-27219 is a selective promoter of the closed confirmation of TG2 and that this leads to enhanced endothelial vasodilatory function by opening of potassium channels as compared to specific inhibitors of TG2 that lock the enzyme in the open conformation and which show contrary effects on the vascular system, see Figure 5 E).

### Example 5 - A pharmacokinetic study.

Using a non-compartmental model, pharmacokinetic studies of a single 2 mg/kg intravenous dose of LDN-27219 in living mice, revealed that the compound remains in the blood for enough time to be adequate for long-term treatments. The estimated values are reported in the following table.

| **Compound** | **Sample size (no of mice)** | **Volume of distribution (L/kg)** | **Half-life (h)** | **Clearance (mL/min·kg)** |
|---|---|---|---|---|
| LDN-27219 | 3 | 21.4 | 4.89 | 52.3 |

### Example 6 - Translatability of murine results to human tissue.

Small human subcutaneous arteries were obtained from fat biopsies from the gluteal region of hypertensive patients and prepared as described in Example 1. Concentration-response curves for LDN-27219 were constructed as described in Example 4 (figure 5B) and relaxation measurements were performed as described in Example 1. The results of the isometric tension studies are shown in Figure 6 A) and B).

This example demonstrates the translatability of the results obtained in murine arteries to human tissue.

### Example 6 - Effect of long-term 'in vivo' treatment with LDN-27219 in diabetic mice compared to reference treatment.

In these experiments, age-paired healthy control mice and diabetic mice were used and the relaxation measurements were performed as described in Example 1. Mesenteric arteries from diabetic male BKS(D)-Leprdb/JOrlRj homozygous mice of 10-11 weeks of age were tested after three weeks of treatment with either 8 mg/kg LDN-27219, vehicle or 3 mg/kg candesartan cilexetil. Arteries from healthy male heterozygous mice were also tested after three weeks of treatment with vehicle as a healthy control. The results of the isometric tension studies are shown in Figure 7.

Example 6 demonstrates that long-term *'in vivo'* treatment with LDN-27219 is able to prevent diabetes-related endothelial dysfunction better than the long-term treatment with angiotensin II receptor antagonist candesartan cilexetil.

### Summary of results and conclusion

Transamidase activity of tissue transglutaminase (TG2), linked to its open conformation, is associated with a series of diseases including cardiovascular disease. Also, TG2 can adopt a closed conformation, presenting GTP binding activity and playing a role in transmembrane signaling. Besides the ability of TG2 inhibitors to inhibit transamidase activity, conformational changes of the enzyme may play a role in their impact on disease. It has been found that general inhibitors (e.g. cystamine) of transglutaminase causes smooth muscle vasodilation. However, it is unclear how these inhibitors affect TG2 conformation and how these different conformations are linked with vascular function.

The present study hypothesized that the closed conformation of TG2 will be associated with vasodilation and antihypertensive effects and may restore age-related changes in endothelial function. It was tested whether LDN-27219 could promote the closed conformation of TG2 and provide improved effects in endothelial function.

Here '*ex vivo'* measurements of vessel tension were performed using isometric myographs in small mesenteric arteries from male Wistar rats, male diabetic healthy control mice and patch-clamp studies in isolated VSMCs. These experiments revealed that induction of the closed conformation of TG2 by LDN-27219 had a direct vasodilatory effect and potentiated the arterial response to acetylcholine through the opening of potassium channels. In contrast, drugs promoting the open conformation of TG2 had no effect in vessel tension and decreased potassium current and acetylcholine response. *'In vivo'* measurements of blood pressure and heart rate revealed that jugular infusion of LDN-27219 induced a drop in blood pressure that was larger in older animals, this was associated with increased expression of TG2 and restoration of endothelial function in isolated arteries from older animals and diabetic animals. Pharmacological modulation of TG2 to its closed conformation using LDN-27219 presents antihypertensive effects *'in vivo'* and potentiates endothelium-dependent vasodilatation by opening potassium channels, increasing these effects with the age of the animal and in diabetic animals. Our findings suggest that the promotion of TG2 closed conformation using LDN-27219 could be a potential strategy to restore age-associated changes in endothelial function as well as diabetes associated changes.

### References

- J.W. Keillor et al., Trends in pharmacological sciences, 2015, Vol 36, no 1
- M. Engholm et al., British J. Pharm. (2016), 173, 839-855
- US 8,614,233
- Y.J. Oh et al., Amino Acids, 2017, 49(3), 695-704
- US 2018/0002700 A1
- WO 2014/057266
- US2006/0183759
- McGrath JC, Lilley E (2015). Implementing guidelines on reporting research using animals (ARRIVE etc.): new requirements for publication in BJP. Br J Pharmacol 172: 3189-3193
- Seltzer A., et al., Brain Res., 2004, 1028(1), 9-18).
- Pinkas DM, et al., PLoS Biol, 2007, 5(12), 2788-2796
- Iversen R, et al., Proc. Natl. Acad. Sci., 2014, 111(48), 17146-17151.

## Claims

1. A compound which is a modulator of tissue transglutaminase (TG2) for use in the treatment of endothelial dysfunction,
wherein the modulator of tissue transglutaminase (TG2) is a compound of formula (I): 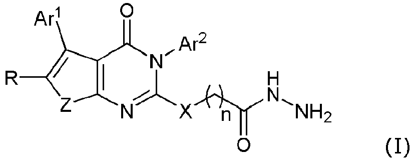 wherein
- Z is selected from the group consisting of S, SO₂, and Se,
- Ar¹ and Ar² are independently selected from the group consisting of optionally substituted phenyl and optionally substituted heteroaryl,
- R is selected from the group consisting of hydrogen, deuterium, halogen, and C₁-C₃ alkyl,
- X is selected from the group consisting of O, NH, N(Me), and S,
n is an integer selected from the group consisting of 1, 2 and 3.

2. The compound for use according to claim 1, wherein the modulator of tissue transglutaminase (TG2) is for use in the treatment of diseases resulting from endothelial dysfunction.

3. The compound for use according to claim 2, wherein the disease resulting from endothelial dysfunction is selected from the group consisting of dementia, Alzheimer's disease, ventricular hypertrophy, vascular calcification, renal fibrosis, cardiac fibrosis, pulmonary fibrosis, atherosclerosis, ischemic chronic wounds, hypertension, pulmonary hypertension, and erectile dysfunction.

4. The compound for use according to claim 3, wherein the disease resulting from endothelial dysfunction is selected from the group consisting of hypertension and erectile dysfunction.

5. The compound for use according to any of the preceding claims, wherein the modulator of tissue transglutaminase (TG2) is a promoter of the closed conformation of tissue transglutaminase.

6. The compound for use according to any of the preceding claims, wherein Z is S.

7. The compound for use according to any of the preceding claims, wherein the optional substituents of Ar¹ and Ar² are selected from the group consisting of C₁-C₁₀ alkyl, C₂-C₁₀ alkenyl, C₂-C₁₀ alkynyl, amino (-NH₂), -CH₂NH(C₁-C₁₀ alkyl), -CH₂N(C₁-C₁₀ alkyl)₂, aminoalkyl (-NH(C₁-C₁₀ alkyl) or -N(C₁-C₁₀ alkyl)₂, cyano (-CN), -CONH₂, -CONH(C₁-C₁₀ alkyl), -CON(C₁-C₁₀ alkyl)₂, hydroxyl (-OH), C₁-C₁₀ alkyl hydroxyl (-alkyl-OH), C₁-C₁₀ alkoxy(-O-alkyl), carboxylic acid (-COOH), C₁-C₁₀ alkyl esters (-COO-alkyl), C₁-C₁₀ alkyl acyl (-CO-alkyl), sulfonic acid (-SO₃H), C₁-C₁₀ alkyl sulfonate (-SOs-alkyl), phosphonic acid (-PO(OH)₂), C₁-C₁₀ alkyl phosphonate (-PO(O-alkyl)₂), phosphinic acid (-P(O)(H)OH), SO₂NH₂, hydroxamic acid (-CONHOH), C₁-C₁₀ alkyl sulfonylureas (-NHCONHSO₂(alkyl)), C₁-C₁₀ acylsulfonamides (-SO₂-NHCO-(alkyl), hydroxyl amine (-NHOH), nitro (-NO₂), and halogen.

8. The compound for use according to any of the preceding claims, wherein
- Ar¹ and Ar² are optionally substituted phenyl,
- R is hydrogen or halogen,
- X is S,
- n is an integer selected from the group consisting of 1, 2 and 3.

9. The compound for use according to any of the preceding claims, wherein the modulator of tissue transglutaminase (TG2) is a compound of formula (II):

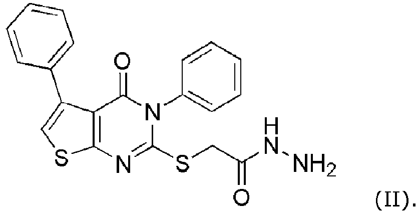

10. The compound for use according to any of the preceding claims, wherein the modulator of tissue transglutaminase (TG2) is LDN-27219.

11. The compound for use according to any of the preceding claims, wherein the endothelial dysfunction is age-associated.

12. The compound for use according to any of the preceding claims, wherein the compound is administered to human patients between the age of 30-140 years, such as the age of 35-140 years, 40-140 years, 45-140 years, 50-140 years, 55-140 years, 60-140 years, 65-140 years, 70-140 years, such as preferably 30-120 years, 40-100 years, such as most preferably 50-100 years.

13. The compound for use according to any of the preceding claims, wherein the endothelial dysfunction is diabetes-associated.

14. The compound for use according to any of the preceding claims, wherein the endothelial dysfunction is associated with diabetes-1 or diabetes-2, preferably diabetes-2.

15. The compound for use according to any of the preceding claims, wherein the endothelial dysfunction is both age-associated and diabetes-associated.

16. The compound for use according to any of the preceding claims, wherein the compound is administered in a composition further comprising a pharmaceutically acceptable carrier or diluent.

## Patentansprüche

1. Verbindung, die ein Modulator von Gewebetransglutaminase (TG2) ist, zur Verwendung bei der Behandlung von endothelialer Dysfunktion,
wobei der Modulator von Gewebetransglutaminase (TG2) eine Verbindung der Formel (I) ist: 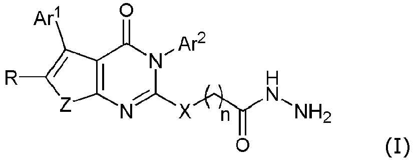 wobei
- Z aus der Gruppe bestehend aus S, SO₂ und Se ausgewählt ist,
- Ar¹ und Ar² unabhängig voneinander aus der Gruppe bestehend aus optional substituiertem Phenyl und optional substituiertem Heteroaryl ausgewählt sind,
- R aus der Gruppe bestehend aus Wasserstoff, Deuterium, Halogen und C₁-C₃-Alkyl ausgewählt ist,
- X aus der Gruppe bestehend aus O, NH, N(Me) und S ausgewählt ist,
n eine ganze Zahl ist, die aus der Gruppe bestehend aus 1, 2 und 3 ausgewählt ist.

2. Verbindung zur Verwendung nach Anspruch 1, wobei der Modulator von Gewebetransglutaminase (TG2) zur Verwendung bei der Behandlung von Erkrankungen ist, die aus endothelialer Dysfunktion resultieren.

3. Verbindung zur Verwendung nach Anspruch 2, wobei die Erkrankung, die aus endothelialer Dysfunktion resultiert, aus der Gruppe bestehend aus Demenz, Alzheimer-Krankheit, ventrikulärer Hypertrophie, Gefäßverkalkung, Nierenfibrose, Herzfibrose, Lungenfibrose, Atherosklerose, ischämischen chronischen Wunden, Hypertonie, pulmonaler Hypertonie und erektiler Dysfunktion ausgewählt ist.

4. Verbindung zur Verwendung nach Anspruch 3, wobei die Erkrankung, die aus endothelialer Dysfunktion resultiert, aus der Gruppe ausgewählt ist, die aus Hypertonie und erektiler Dysfunktion besteht.

5. Verbindung zur Verwendung nach einem der vorhergehenden Ansprüche, wobei der Modulator von Gewebetransglutaminase (TG2) ein Promotor der geschlossenen Konformation von Gewebetransglutaminase ist.

6. Verbindung zur Verwendung nach einem der vorhergehenden Ansprüche, wobei Z S ist.

7. Verbindung zur Verwendung nach einem der vorhergehenden Ansprüche, wobei die optionalen Substituenten von Ar¹ und Ar² aus der Gruppe ausgewählt sind, die aus C₁-C₁₀-Alkyl, C₂-C₁₀-Alkenyl, C₂-C₁₀-Alkinyl, Amino (-NH₂), -CH₂NH(C₁-C₁₀-Alkyl), -CH₂N(C₁-C₁₀-Alkyl)₂, Aminoalkyl (-NH(C₁-C₁₀-Alkyl) oder -N(C₁-C₁₀-Alkyl)₂, Cyano (-CN), -CONH₂, -CONH(C₁-C₁₀-Alkyl), -CON(C₁-C₁₀-Alkyl)₂, Hydroxyl (-OH), C₁-C₁₀-Alkylhydroxyl (-Alkyl-OH), C₁-C₁₀-Alkoxy (-O-Alkyl), Carbonsäure (-COOH), C₁-C₁₀-Alkylester (-COO-Alkyl), C₁-C₁₀-Alkylacyl (-CO-Alkyl), Sulfonsäure (-SO₃H), C₁-C₁₀-Alkylsulfonat (-SO₃-Alkyl), Phosphonsäure (-PO(OH)₂), C₁-C₁₀-Alkylphosphonat (-PO(O-Alkyl)₂), Phosphinsäure (-P(O)(H)OH), SO₂NH₂, Hydroxamsäure (-CONHOH), C₁-C₁₀-Akylsulfonylharnstoffe (-NHCONHSO₂(Alkyl)), C₁-C₁₀-Acylsulfonamide (-SO₂-NHCO-(Alkyl), Hydroxylamin (-NHOH), Nitro (-NO₂) und Halogen besteht.

8. Verbindung zur Verwendung nach einem der vorhergehenden Ansprüche, wobei
- Ar¹ und Ar² optional substituiertes Phenyl sind,
- R Wasserstoff oder Halogen ist,
- X S ist,
- n eine ganze Zahl ist, die aus der Gruppe bestehend aus 1, 2 und 3 ausgewählt ist.

9. Verbindung zur Verwendung nach einem der vorhergehenden Ansprüche, wobei der Modulator von Gewebetransglutaminase (TG2) eine Verbindung der Formel (II) ist:

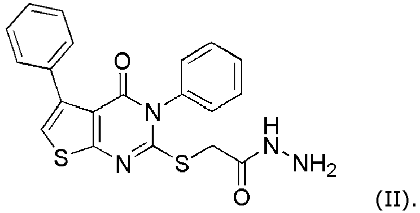

10. Verbindung zur Verwendung nach einem der vorhergehenden Ansprüche, wobei der Modulator von Gewebetransglutaminase (TG2) LDN-27219 ist.

11. Verbindung zur Verwendung nach einem der vorhergehenden Ansprüche, wobei die endotheliale Dysfunktion altersassoziiert ist.

12. Verbindung zur Verwendung nach einem der vorhergehenden Ansprüche, wobei die Verbindung menschlichen Patienten im Alter von 30-140 Jahren, wie etwa im Alter von 35-140 Jahren, 40-140 Jahren, 45-140 Jahren, 50-140 Jahren, 55-140 Jahren, 60-140 Jahren, 65-140 Jahren, 70-140 Jahren, wie etwa bevorzugt 30-120 Jahren, 40-100 Jahren, wie etwa am bevorzugtesten 50-100 Jahren verabreicht wird.

13. Verbindung zur Verwendung nach einem der vorhergehenden Ansprüche, wobei die endotheliale Dysfunktion diabetesassoziiert ist.

14. Verbindung zur Verwendung nach einem der vorhergehenden Ansprüche, wobei die endotheliale Dysfunktion mit Diabetes-1 oder Diabetes-2, bevorzugt Diabetes-2, assoziiert ist.

15. Verbindung zur Verwendung nach einem der vorhergehenden Ansprüche, wobei die endotheliale Dysfunktion sowohl altersassoziiert als auch diabetesassoziiert ist.

16. Verbindung zur Verwendung nach einem der vorhergehenden Ansprüche, wobei die Verbindung in einer Zusammensetzung verabreicht wird, die ferner einen pharmazeutisch verträglichen Träger oder ein pharmazeutisch verträgliches Verdünnungsmittel umfasst.

## Revendications

1. Composé qui est un modulateur de la transglutaminase tissulaire (TG2) destiné à être utilisé dans le traitement d'un dysfonctionnement endothélial,
dans lequel le modulateur de la transglutaminase tissulaire (TG2) est un composé de formule (I) : 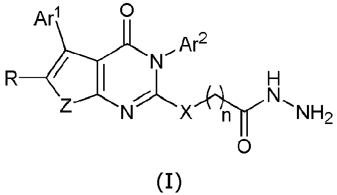 dans laquelle
- Z est sélectionné dans le groupe comprenant S, SO₂, et Se,
- Ar¹ et Ar² sont sélectionnés indépendamment dans le groupe constitué de phényle éventuellement substitué et hétéroaryle éventuellement substitué,
- R est sélectionné dans le groupe constitué d'hydrogène, deutérium, halogène et alkyle en C₁ à C₃,
- X est sélectionné dans le groupe constitué de O, NH, N(Me), et S,
n est un nombre entier sélectionné dans le groupe constitué de 1, 2 et 3.

2. Composé destiné à être utilisé selon la revendication 1, dans lequel le modulateur de la transglutaminase tissulaire (TG2) est destiné à être utilisé dans le traitement de maladies résultant d'un dysfonctionnement endothélial.

3. Composé destiné à être utilisé selon la revendication 2, dans lequel la maladie résultant d'un dysfonctionnement endothélial est sélectionnée dans le groupe constitué de la démence, la maladie d'Alzheimer, l'hypertrophie ventriculaire, la calcification vasculaire, la fibrose rénale, la fibrose cardiaque, la fibrose pulmonaire, l'athérosclérose, les plaies chroniques ischémiques, l'hypertension, l'hypertension pulmonaire et le dysfonctionnement érectile.

4. Composé destiné à être utilisé selon la revendication 3, dans lequel la maladie résultant d'un dysfonctionnement endothélial est sélectionnée dans le groupe constitué de l'hypertension et le dysfonctionnement érectile.

5. Composé destiné à être utilisé selon l'une quelconque des revendications précédentes, dans lequel le modulateur de la transglutaminase tissulaire (TG2) est un promoteur de la conformation fermée de la transglutaminase tissulaire.

6. Composé destiné à être utilisé selon l'une quelconque des revendications précédentes, dans lequel Z est S.

7. Composé destiné à être utilisé selon l'une quelconque des revendications précédentes, dans lequel les substituants facultatifs de Ar¹ et Ar² sont sélectionnés dans le groupe constitué de alkyle en C₁ à C₁₀, alcényle en C₂ à C₁₀, alcynyle en C₂ à C₁₀, amino (-NH₂), -CH₂NH(alkyle en C₁ à C₁₀), -CH₂N(alkyle en C₁ à C₁₀)₂, aminoalkyle (-NH(alkyle en C₁ à C₁₀) ou -N(alkyle en C₁ à C₁₀)₂, cyano (-CN), -CONH₂, -CONH(alkyle en C₁ à C₁₀), -CON(alkyle en C₁ à C₁₀)₂, hydroxyle (-OH), hydroxyle alkyle en C₁ à C₁₀ (-alkyl-OH), alcoxy en C₁ à C₁₀(-O-alkyle), acide carboxylique (-COOH), esters alkyle en C₁ à C₁₀ (-COO-alkyl),acyl alkyle en C₁ à C₁₀ (-CO-alkyl), acide sulfonique (-SO₃H), sulfonate d'alkyle en C₁ à C₁₀ (-SO₃-alkyle), acide phosphonique (-PO(OH)₂), phosphonate d'alkyle en C₁ à C₁₀ (-PO(O-alkyle)₂), acide phosphinique (-P(O)(H)OH), SO₂NH₂, acide hydroxamique (-CONHOH), sulfonylurées alkyle en C₁ à C₁₀ (-NHCONHSO₂(alkyle)), acylsulfonamides en C₁ à C₁₀ (-SO2-NHCO-(alkyle), hydroxylamine (-NHOH), nitro (-NO₂) et halogène.

8. Composé selon l'une quelconque des revendications précédentes, dans lequel
- Ar¹ et Ar² sont phényle éventuellement substitué,
- R est hydrogène ou halogène ;
- X est S,
- n est un nombre entier sélectionné dans le groupe constitué de 1, 2 et 3.

9. Composé destiné à être utilisé selon l'une quelconque des revendications précédentes, dans lequel le modulateur de la transglutaminase tissulaire (TG2) est un composé de formule (II) :

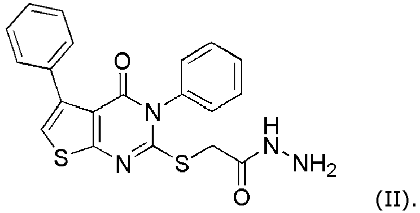

10. Composé destiné à être utilisé selon l'une quelconque des revendications précédentes, dans lequel le modulateur de la transglutaminase tissulaire (TG2) est le LDN-27219.

11. Composé destiné à être utilisé selon l'une quelconque des revendications précédentes, dans lequel le dysfonctionnement endothélial est associé à l'âge.

12. Composé destiné à être utilisé selon l'une quelconque des revendications précédentes, dans lequel le composé est administré à des patients humains âgés de 30 à 140 ans, tels que âgés de 35 à 140 ans, 40 à 140 ans, 45 à 140 ans, 50 à 140 ans, 55 à 140 ans, 60 à 140 ans, 65 à 140 ans, 70 à 140 ans, tels que de préférence de 30 à 120 ans, 40 à 100 ans, tels que le plus préférablement de 50 à 100 ans.

13. Composé destiné à être utilisé selon l'une quelconque des revendications précédentes, dans lequel le dysfonctionnement endothélial est associé au diabète.

14. Composé destiné à être utilisé selon l'une quelconque des revendications précédentes, dans lequel le dysfonctionnement endothélial est associé au diabète-1 ou au diabète-2, de préférence au diabète-2.

15. Composé destiné à être utilisé selon l'une quelconque des revendications précédentes, dans lequel le dysfonctionnement endothélial est à la fois associé à l'âge et associé au diabète.

16. Composé destiné à être utilisé selon l'une quelconque des revendications précédentes, dans lequel le composé est administré dans une composition comprenant en outre un vecteur ou un diluant pharmaceutiquement acceptable.
